Europäisches Patentamt

European Patent Office

Office européen des brevets

(11) Publication number: **0 408 757 B1**

# EUROPEAN PATENT SPECIFICATION

(45) Date of publication of patent specification: **29.03.95** (51) Int. Cl.$^6$: **A61B 17/36**

(21) Application number: **90900333.7**

(22) Date of filing: **12.12.89**

(86) International application number:
**PCT/JP89/01243**

(87) International publication number:
**WO 90/06727 (28.06.90 90/15)**

(54) **LASER BEAM TRANSMITTING MEMBER AND METHOD OF MANUFACTURING THE SAME.**

(30) Priority: **12.12.88 JP 313305/88**

(43) Date of publication of application:
**23.01.91 Bulletin 91/04**

(45) Publication of the grant of the patent:
**29.03.95 Bulletin 95/13**

(84) Designated Contracting States:
**AT CH DE ES FR GB IT LI NL SE**

(56) References cited:
**GB-A- 2 154 761**
**JP-A-59 120 148**
**JP-A-63 318 934**
**JP-U-62 183 814**
**US-A- 4 736 743**

(73) Proprietor: **S.L.T. JAPAN CO, LTD.**
**3rd Floor Tateno Bldg., 15-5**
**Iidabashi 2-chome, Chiyoda-ku**
**Tokyo 102 (JP)**

(72) Inventor: **DAIKUZONO, Norio**
**381, Kofudai 4-chome**
**Ichihara-shi**
**Chiba-ken 290-02 (JP)**

(74) Representative: **Bloch, Gérard et al**
**2, square de l'Avenue du Bois**
**F-75116 Paris (FR)**

Rank Xerox (UK) Business Services
(3.10/3.09/3.3.3)

**Description**

This invention relates to a laser light transmissible article such as a laser light emitter which permits, for living tissue of an animal such as a human body, an incision and vaporization of the living tissue, a thermal therapy, and the like. Further, this invention relates to a method for producing the laser light transmissible article.

Medical treatments such as incisions of living tissue of animal organisms by irradiation with laser light are conspicuous due to its ability of hemostasis in these days.

It had been the conventional method that the laser light was irradiated from the fore end of an optical fiber system which is brought out of contact with the living tissue (hereafter "living tissue" is sometimes expressed by "tissue" only). But this method causes severe damage to the fore end of the optical fiber system. Therefore, a method which has been utilized lately is as follows; at first, the laser light is transmitted into an optical fiber. Then, the laser light is fed into an emitting probe, which is brought into or out of contact with the tissue being adjacent to the fore end of the optical fiber. Finally, the laser light emitted from the surface of the probe is irradiated on the tissue.

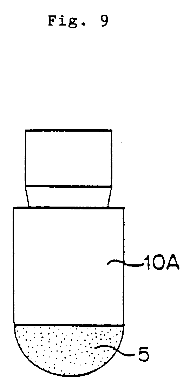

The inventor developed many kinds of contact probes which are utilized for various purposes. One embodiment is shown in Fig. 8. This probe is made of sapphire, quartz and the like, and usually, its surface is smooth.

Referring to Fig. 8, laser light L is fed by means of an optical fiber 51 into the probe 50, which is of long and narrow conical shape with a round tip end and whose external surface is smooth. The laser light L passing through the probe 50 is reflected and refracted on an inner surface to reach the tip end, finally is emitted only from the tip end.

In this case, a power density of the laser light L is shown as contour lines H. Then, when the probe 50 is brought into contact with the tissue, the distribution of the power density in a longitudinal direction in the tissue is shown as a curve Pd. Accordingly, it is obvious that the laser light L is concentratedly emitted from the tip end of the probe 50. Therefore, the effective area of laser light irradiation is significantly small.

Under these circumstances, as shown in Fig. 10, the inventor found that forming a rough surface 50a on the external surface of the probe 50A extends the effective area of laser light irradiation, because the laser light is refracted on the rough surface 50a to be emitted in many directions. A surface layer 5A made of a light transmissible material is formed on the rough surface 50a for refraction of the laser light.

Although it is possible to extend the effective area of the laser light irradiation by means of this method, the efficiency is not sufficient coupled with following defects.

First, in case of the incision of less hemorrhagic tissue such as skin or fat layer, this treatment causes a severe damage to the tissue, and further requires a high power level of the laser light. This necessitates a high power and expensive laser light generator. Further, since the probe must be moved slowly, a medical treatment using this conventional probe can not be carried out quickly.

Secondly, the material and form (especially form) of this conventional probe and the required power level of the laser light should be changed corresponding to each type of a target area of the tissue and each type of the medical treatment. In other words, it is difficult to apply this conventional probe for various types of medical treatments.

From US-A-4 736 743, it is also known to add light absorbing particles to the rough surface of the probe. However, as the amount of these absorbing particles, the incision is carried mainly by evaporation and the depth of the coagulation is reduced.

It is therefore an object of the present invention to provide a laser light transmissible article which has the large effective area of laser light irradiation, which has the high efficiency of an incision by tissue vaporization and hemostasis, which requires a low power level of laser light and can be moved quickly as a probe in case of an incision for less hemorrhagic tissue and which can be used as a suitable probe for every type of medical treatment and every type of target area by easy selection of the probe from the same shaped several kinds of probes differing in only the content of laser light absorbing particles and light scattering particles. Further, another object of the present invention is to provide a producing method of this laser light transmissible article.

In a prefered embodiment, a rough surface is formed on the transmissible member and the surface layer is provided on the rough surface.

The instant invention also relates to a method for producing a laser light transmissible article by forming a surface layer on a laser light transmissible member, characterized by the fact that it comprises the step of bringing said transmissible member in contact with a dispersion including laser light transmissible particles, of a third type, laser light absorbing particles, of a first type, and laser light scattering particles, of a second type, which have a refractive index larger than that of said transmissible member and wherein the particles of the second type A constitute from 70-20 % in weight of the surface layer, the particles of the first type C constitute from 70-10 % in weight of the surface layer, and the particles of the third type B constitute from 20-50% in weight of the surface layer.

## Disclosure of the Invention

To this end, the present invention relates to a laser light transmissible article comprising:

a laser light transmissible member; and

a surface layer covering a surface of said laser light transmissible member, characterized by the fact that

said surface layer contains

(i) particles of a first type C which absorb laser light,

(ii) particles of a second type A which scatter laser light, said second type A of particles being different from said first type C of particles and having a refractive index larger than that of said transmissible member,

(iii) particles of a third type B which are transmissible to laser light and act as a binder for the particles of the first and second type C, A, said third type B of particles being different from said first type of particles and said second type of particles, wherein :

the particles of the second type A constitute from 70-20 % in weight of the surface layer,

the particles of the first type C constitute from 70-10 % in weight of the surface layer, and

the particles of the third type B constitute from 20-50 % in weight of the surface layer.

In a prefered embodiment, a rough surface is formed on the transmissible member and the surface layer is provided on the rough surface.

The instant invention also relates to a method for producing a laser light transmissible article by forming a surface layer on a laser light transmissible member, characterized by the fact that it comprises the step of bringing said transmissible member in contact with a dispersion including laser light transmissible particles, of a third type, laser light absorbing particles, of a first type, and laser light scattering particles, of a second type, which have a refractive index larger than that of said transmissible member and wherein the particles of the second type A constitute from 70-20 % in weight of the surface layer the particles of the first type C constitute from 70-10 % in weight of the surface layer, and the particles of the third type B constitute from 20-50 % in weight of the surface layer.

Preferably, said laser light transmissible particles have a melting point identical to or lower than that of said transmissible member and the transmissible member is baked with the dispersion at a temperature higher than the melting point of said transmissible particles and at a limit temperature enabling said transmissible member to keep its shape.

It should be pointed out that GB-A-2 154 761 teaches a laser probe for phototherapy of tumours comprising scattering particles for efficiently diffusing transmitted light. No mention, however, is made of their coagulating effect.

Now, the present invention is described more particularly by way of the embodiments shown in the drawings.

## Brief Description of the Drawings

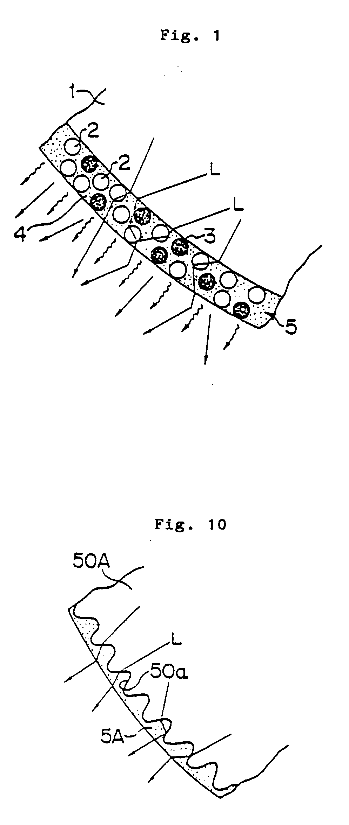

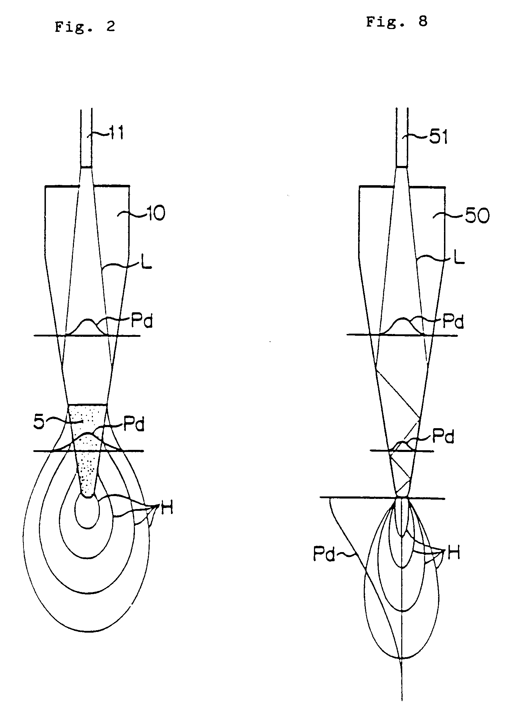

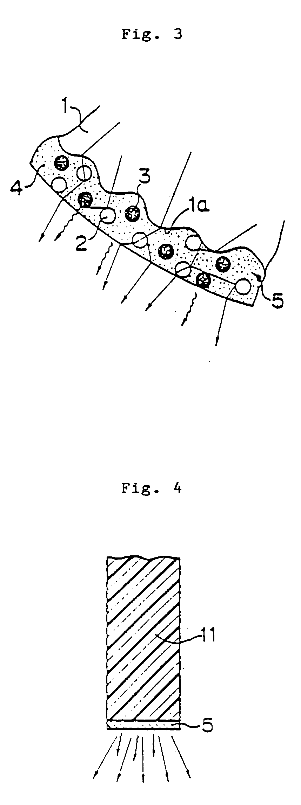

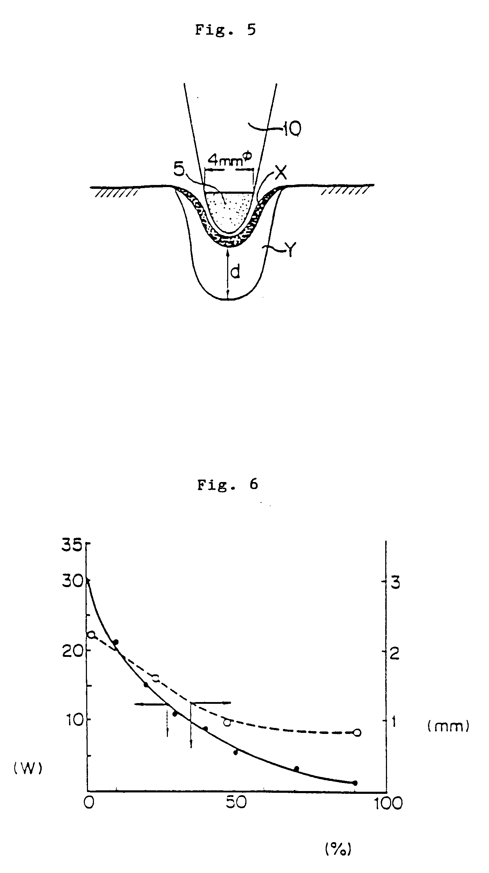

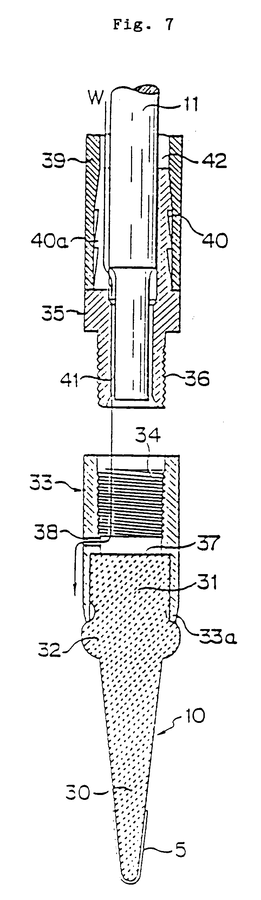

Fig. 1 is an enlarged sectional view of a surface layer of the present invention; Fig. 2 is a schematic illustration showing an embodiment of a laser light transmissible article as a probe for a laser scalpel and a power density distribution diagram of laser light emitted from this probe; Fig. 3 is an enlarged sectional view of a surface layer formed on a rough surface of a laser light transmissible material; Fig. 4 is a sectional view of a surface layer on an optical fiber; Fig. 5 is a schematic illustration explaining an experiment of an incision of living tissue with a probe; Fig. 6 is a graph showing the result of the experiment illustrated in Fig. 5; Fig. 7 is a longitudinal sectional view of an example of a structure of a probe and a holding member therefor; Fig. 8 is a schematic illustration showing an embodiment of a conventional probe and a power density distribution diagram of laser light with this conventional probe; Fig. 9 is an elevational view of a probe having another shape; Fig. 10 is an enlarged sectional view of a surface of a conventional probe.

According to the present invention, as shown in Fig. 1, the transmissible member 1 is provided on its surface with the surface layer 5 which contains the light scattering particles 2 made of sapphire and the like having a larger refractive index than that of the transmissible member 1. While the laser light L emitted from the transmissible member 1 passes through the surface layer 5, the laser light L impinges on the light scattering particle 2 in the surface layer 5 to be partially reflected on the surface of the particle 2, or to be partially penetrated into and emitted from the particle 2 with refraction. Therefore, the laser lights L is emitted in various directions from the whole external surface of the surface layer 5. This produces the large area of the laser light irradiation.

Further, the surface layer 5 contains the laser light absorbing particles 3 made of carbon and the like. Accordingly, when the laser light L impinges on the laser light absorbing particle 3, the greater part of the energy of the laser light L is converted to heat energy by means of the laser light absorbing particle 3, and the tissue is heated by the heat energy from the surface layer 5.

By so doing, as the vaporization of the tissue is accelerated, the tissue can be incised with a low energy of the laser light L penetrated into the transmissible member 1. Therefore, when the tissue is incised, the transmissible member 1 can be moved quickly.

Accordingly, the medical treatment can be carried out in short time. Further, since high power level of the laser light L is not required, the medical treatment can be carried out with a cheap and small scaled laser light generator.

On the other hand, as for the producing method of the surface layer, if a dispersion containing the laser light absorbing particles and the light scattering particles is coated on the surface of the transmissible member, after a vaporization of a dispersion medium, the contact of the laser light transmissible substance serving as a probe having the surface layer with the tissue or other substances causes a damage to the surface layer. Because, the both particles are attached to the surface of the transmissible member only by physical adsorptive power.

Therefore, by a binder which sticks the laser light absorbing particles and the light scattering particles to the surface of the transmissible member, an adhesion of the surface layer to the transmissible member is enhanced.

In this case, the binder is preferably made of a light transmissible material 4 such as quartz and the like to ensure the emitting of the laser light from the surface layer 5. As the transmissible material 4, laser light transmissible particles having a melting point same to or lower than that of the transmissible member 1 are used and they are dispersed together with the absorbing particles and the light scattering particles in a proper liquid such as water and alcohol. Then the transmissible member 1 painted with this dispersion is baked at a higher temperature than a melting point of the transmissible particle and within a limit temperature so that the transmissible member 1 can keep its shape. Accordingly, the transmissible particles melt to form the surface layer of high mechanical strength together with the laser light absorbing particles and the light scattering particles. Therefore, the damages to the surface layer can be reduced because of its high strength.

The surface layer 5 contains laser the light scattering particles 2 and the laser light absorbing particles 3 with laser light transmissible particles which are melted to be a laser light transmissible material 4 as binder. Further, as shown in Fig. 3, if a rough surface 1a is formed on the transmissible member 1 and the surface layer 5 is provided on the rough surface, the scattering effect of the laser light is increased.

The probe 10, for example, is held by a holding member shown in Fig. 7.

This probe 10 comprises a tapered conical inserting portion 30, a main holding portion 31 and a flange 32 formed between them. The probe 10 is fitted in a cylindrical female connector 33 and fixed integrally thereto by caulking the mating portions 33a and/or by using a ceramic type adhesive between the mating surfaces. The female connector 33 has, on its internal surface, a female thread 34 which is adapted to mate removably with male threads 36 of a male connector 35. The female connector 33 has holes 38 which facilitate the passage of cooling water W inside and outside thereof. The holes 38 are disposed to be adjacent to the top of a light receiving base 37 of the probe 10 and, for example, oppositely on the circumference of the female connector 33, although only one of them is shown in Fig. 7

On the other hand, the male connector 35 is pressed to be fitted into an end portion of a flexible tube jacket 39 fabricated of, for example, polytetrafluoroethylene such as Teflon (trademark). For this press fitting, the male connector 35 has stepped portions 40 at its base portion in order to be firmly held by the tube jacket 39 so as not to be removed.

A transmitting optical fiber 11 for the laser light is inserted in the tube jacket 39 and the male connector 35. There is a gap 42 between the optical fiber 11 and the tube jacket 39 for supplying cooling water. The fore end portion of the transmitting optical fiber 11 is closely fitted in the male connector 35 at its stepped portion 40, however, the stepped portion 40 has, for example, two slits 40a formed oppositely on the circumference of the stepped portion 40 for letting the cooling water W pass through. A passage 41 for the cooling water W is further provided between the inner surface of the fore end portion of the male connector 35 and the external surface of the transmitting optical fiber 11.

This probe held by the above mentioned holding member serves as a laser light emitter while the female connector 33 is connected to mate with the male connector 35. In this way, the emitter is equipped in an endoscope or some other suitable holders. Then, pulse laser light introduced through the transmitting optical fiber 11 penetrates into the probe 10 from the light receiving base 37, therefore, is emitted from all over the external surface of the inserting portion 30. At the same time, the cooling water W is fed through a gap 42, the slit 40a and a passage 41 to cool the probe 10, further, discharged through the opening 38 to flow out on the surface of the tissue so as to cool it.

The transmissible member of this invention is preferably fabricated from a natural or artificial ceramic material such as diamond, sapphire, quartz and the like due to their heat resistance.

The light scattering particle, which has a larger refractive index for the laser light than that of the transmissible member, is of a natural or artificial material such as diamond, sapphire, quartz (a melting point is preferably high), single crystal zirconium oxide ($ZrO_2$), high melting point glass, transmissible and heat resistant synthetic resin, laser light reflective metal, and a particle which is laser light reflective or non-reflective metal particle coated with laser light reflective metal such as gold, aluminum and the like by means of a surface treatment such as gilding.

In order to form the surface layer surely, the laser light transmissible material of the binder is preferably made from the transmissible particle, which can be melt to form a film and more preferably which has heat resistance, such as natural or artificial sapphire, quartz, glass, transmissible and heat resistant synthetic resin and the like. A suitable transmissible material is selected from these materials in consideration of the relation to the transmissible member.

The laser light absorbing particle is made of carbon, graphite, iron oxide, manganese dioxide and any other materials which can absorb the laser light to generate heat energy.

A content of each particle in the surface layer(wt%) and each average particle size are preferably within ranges as shown in a following table respectively. More preferable content and particle size are put in parentheses.

|  | Content(wt%) | Average Particle Size($\mu$m) |
| --- | --- | --- |
| Light Scattering Particle(A) | 90 - 1 (70 - 20) | 0.2-300 (1 - 50) |
| Transmissible Particle(B) | 10 - 90 (20 - 50) | 0.2 - 500 |
| Absorbing Particle(C) | 90 - 1 (70 - 10) | 0.2 - 500 (1 - 100) |

The thickness of the surface layer is preferably 10$\mu$m - 5mm, more preferably 30$\mu$m - 1mm. The surface layer is formed by one of following methods. If the surface layer can not be formed to be a desired thickness for this once, the operation of the method should be repeated until a desired thickness can be obtained;

First method; the three kinds of particles are dispersed in a dispersion medium. Then the medium is heated to a temperature which is higher than the melting point of the transmissible particle. Finally, the transmissible member is dipped in the heated dispersion.

Second method; the three kinds of particles are melted to be sprayed to the transmissible member.

Further, other suitable methods for forming the surface layer can be used. However, above all, the first method facilitates operation, because what should be done is that only a part of the transmissible member, which is desired to be the surface layer, necessitates being dipped in the dispersion and therefrom pulled up. That is to say, the first method is practical and rational.

As for the dispersion medium, suitable liquid such as water, alcohol or mixture of them can be used. Further sugar or starch is added to increase the viscosity of the dispersion medium.

As described above, according to the present invention, the forming of the surface layer 5 on the surface of the transmissible member 1 extends the effective area of laser light irradiation, because, the laser light is emitted widely in many directions from the surface layer 5 as shown in Fig. 2.

On the other hand, the inventor performed an experiment as follows, using a probe as shown in Fig. 5;

In a following section, the content of the light scattering particle, that of the transmissible particle, and that of the laser light absorbing particle will be referred as (A), (B) and (C) respectively. The inventor investigated each change of following two parameters against (C) under fixed condition of (A) : (B) = 2 : 1. One investigated parameter is a laser light power level with which an incision to a pig's liver can be started. Another is a depth d of a coagulation layer Y below a carbonized layer X. Then, the result of this experiment is obtained as shown in Fig. 6. Then, following things are known.

According to the relation of the power level and the percentage of (C), when the percentage of (C) is high, the incision can be started with the low power level of the laser light, then it is possible for the probe to be moved quickly. Then, according to the relation of the depth d and the percentage of (C), as the percentage of (C) is increased, the depth d is reduced. Since the effect of hemostasis can be known by the depth d, it is clear that the hemostasis of the treated tissue is reduced as the percentage of (C) is increased.

Therefore, the probe with high percentage of (C) in the surface layer can be used effectively for incising the tissue which bear the damage to some extent such as skin, fat layer and the like.

On the other hand, the probe with low percentage of (C) can be used efficiently for incising the tissue, for which the hemostasis is regarded to be important. This type of the tissue is, for example, liver, heart and the like. In this case, it is also clear that the laser light power level of the output from a laser light generator must be raised and the probe must be moved slowly.

Referring to this experiment and the like, the inventor introduced these two equations, (1) and (2).

$$\frac{(C)}{(A)+(B)+(C)} \propto \frac{Quantity\ of\ laser\ light\ for\ heating}{Incident\ laser\ energy} \qquad (1)$$

$$\frac{(A)+(B)}{(A)+(B)+(C)} \propto \frac{Quantity\ of\ laser\ light\ for\ transmitting}{Incident\ laser\ energy} \qquad (2)$$

Equation (1) means that heat generation is progressed as (C) is increased. Accordingly, under high percentage of (C), an incision is carried out by mainly vaporization. Therefore, since most of the incident laser light energy is spent for the heating, the laser light can not penetrate so deeply into the tissue. As a result, the depth of the coagulation layer is reduced.

Equation (2) means that penetration of the laser light into the tissue is progressed as (C) is decreased. Accordingly, under the low percentage of (C), the tissue absorbing laser light is heated, thus, the coagulation is made in the tissue.

Therefore, if some kinds of probes which differs in only percentage of (C) in the surface layers are prepared in advance, a suitable probe can be selected in accordance with a medical purpose, thereby a suitable treatment can be carried out easily.

The probe of the present invention is not limited to the probe shown in Fig. 2. It may be a cylindrical shaped probe 10A having a hemispherical end and a surface layer 5 thereon as shown in Fig. 9 or another shaped probe. Although it is not shown, it may be a cylindrical shaped probe having a surface layer on the inner surface thereof. Further, an optical fiber 11 might serve as a transmissible member and is covered with a surface layer 5 as shown in Fig. 4.

On the other hand, the laser light transmissible substance of the present invention is applied to a power meter in a thermal therapy, as well as a laser scalpel.

As explained in the foregoing description, the transmissible article of the present invention can be applied effectively when the large effective area of the laser light irradiation for living tissue is required.

## Claims

1. A laser light transmissible article (10) comprising :
   a laser light transmissible member (1, 30); and
   a surface layer (5) covering a surface of said laser light transmissible member (1, 30),
   said surface layer (5) containing
   (i) particles (3) of a first type C which absorb laser light, characterized by the fact that said surface layer further contains
   (ii) particles (2) of a second type A which scatter laser light, said second type A of particles (2) being different from said first type C of particles (3) and having a refractive index larger than that of said transmissible member (1, 30),
   (iii) particles (4) of a third type B which are transmissible to laser light and act as a binder for the particles (3, 2) of the first and second type C, A, said third type B of particles (4) being different from said first type of particles and said second type of particles, wherein :
   the particles (2) of the second type A constitute from 70-20 % in weight of the surface layer (5),
   the particles (3) of the first type C constitute from 70-10 % in weight of the surface layer (5), and
   the particles (4) of the third type B constitute from 20-50 % in weight of the surface layer (5).

6

2. A laser light transmissible article according to claim 1, wherein a rough surface (1a) is formed on said transmissible member (1, 30) and said surface layer (5) is provided on said rough surface (1a).

3. A method for producing a laser light transmissible article (10) by forming a surface layer (5) on a laser light transmissible member (1, 30), characterized by the fact that it comprises the step of bringing said transmissible member (1, 30) in contact with a dispersion including laser light transmissible particles (4), of a third type, laser light absorbing particles (3), of a first type, and laser light scattering particles (2), of a second type, which have a refractive index larger than that of said transmissible member (1, 30) and wherein the particles(2) of the second type A constitute from 70-20 % in weight of the surface layer (5,) the particles (3) of the first type C constitute from 70-10 % in weight of the surface layer (5), and the particles (4) of the third type B constitute from 20-50 % in weight of the surface layer (5).

4. A method according to claim 3, wherein said laser light transmissible particles (4) have a melting point identical to or lower than that of said transmissible member (1, 30) and the transmissible member (1, 30) is baked with the dispersion at a temperature higher than the melting point of said transmissible particles (4) and at a limit temperature enabling said transmissible member (1, 30) to keep its shape.

**Patentansprüche**

1. Für Laser-Licht durchlässiger Gegenstand (10), der enthält:
   ein für Laser-Licht durchlässiges Element (1, 30); und
   eine Oberflächenschicht (5), die eine Fläche des für Laser-Licht durchlässigen Elements (1, 30) bedeckt,
   wobei die Oberflächenschicht (5)
   (i) Teilchen (3) von einer ersten Art C enthält, die Laser-Licht absorbieren,
   dadurch gekennzeichnet, daß die Oberflächenschicht weiters
   (ii) Teilchen (2) von einer zweiten Art A enthält, die Laser-Licht streuen, wobei sich die zweite Art von Teilchen (2) von der ersten Art C von Teilchen (3) unterscheidet und einen Brechungsindex besitzt, der größer als der Brechungsindex des durchlässigen Elements (1, 30) ist, sowie
   (iii) Teilchen (4) von einer dritten Art B enthält, die für Laser-Licht durchlässig sind und als Bindemittel für die Teilchen (3, 2) der ersten und zweiten Art C, A wirken, wobei sich die dritte Art B von Teilchen (4) von der ersten Art von Teilchen und der zweiten Art von Teilchen unterscheidet, wobei:
   die Teilchen (2) von der zweiten Art A 70 - 20 Gewichtsprozent der Oberflächenschicht (5) bilden,
   die Teilchen (3) von der ersten Art C 70 - 10 Gewichtsprozent der Oberflächenschicht (5) bilden, und
   die Teilchen (4) von der dritten Art B 20 - 50 Gewichtsprozent der Oberflächenschicht (5) bilden.

2. Für Laser-Licht durchlässiger Gegenstand gemäß Anspruch 1, wobei auf dem durchlässigen Element (1, 30) eine rauhe Oberfläche (1a) ausgebildet wird, wobei die Oberflächenschicht (5) auf der rauhen Oberfläche (1a) vorgesehen ist.

3. Verfahren zur Herstellung eines für Laser-Licht durchlässigen Gegenstands (10), indem auf einem für Laser-Licht durchlässigen Element (1, 30) eine Oberflächenschicht (5) ausgebildet wird, durch gekennzeichnet, daß das Verfahren einen Schritt enthält, bei dem das durchlässige Element (1, 30) mit einer Dispersion in Berührung gebracht wird, die für Laser-Licht durchlässige Teilchen (4) von einer dritten Art, Laser-Licht absorbierende Teilchen (3) von einer ersten Art sowie Laser-Licht streuende Teilchen (2) von einer zweiten Art aufweist, die einen Brechungsindex besitzen, der größer als der Brechungsindex des durchlässigen Elements (1, 30) ist, und wobei die Teilchen (2) von der zweiten Art A 70 - 20 Gewichtsprozent der Oberflächenschicht (5) bilden, die Teilchen (3) von der ersten Art C 70 - 10 Gewichtsprozent der Oberflächenschicht (5) bilden, und die Teilchen (4) von der dritten Art B 20 - 50 Gewichtsprozent der Oberflächenschicht (5) bilden.

4. Verfahren gemäß Anspruch 3, wobei die für Laser-Licht durchlässigen Teilchen (4) einen Schmelzpunkt besitzen, der genau gleich oder niedriger als der Schmelzpunkt des durchlässigen Elements (1, 30) ist, wobei das durchlässige Element (1, 30) mit der Dispersion bei einer Temperatur, die höher als die Temperatur des Schmelzpunkts der durchlässigen Teilchen (4) ist, sowie bei einer Grenztemperatur gebacken wird, bei der das durchlässige Element (1, 30) seine Form beibehalten kann.

**EP 0 408 757 B1**

**Revendications**

1. Article (10) permettant la transmission d'une lumière laser, comprenant :

   un organe (1, 30) permettant la transmission d'une lumière laser; et

   une couche de surface (5) recouvrant une surface dudit organe (1, 30) permettant la transmission d'une lumière laser,

   ladite couche de surface (5) contenant :

   (i) des particules (3) d'un premier type C qui absorbe la lumière laser, caractérisé par le fait que ladite couche de surface contient en outre :

   (ii) des particules (2) d'un deuxième type A qui disperse la lumière laser, ledit deuxième type A de particules (2) étant différent dudit premier type C de particules (3) et présentant un indice de réfraction supérieur à celui dudit organe (1, 30) permettant la transmission,

   (iii) des particules (4) d'un troisième type B permettant la transmission d'une lumière laser et servant de liant pour les particules (3, 2) des premier et deuxième types C, A, ledit troisième type B de particules (4) étant différent dudit premier type de particules et dudit deuxième type de particules, dans lequel :

   les particules (2) du deuxième type A constituent 70 à 20 % en poids de la couche de surface (5),

   les particules (3) du premier type C constituent 20 à 50 % en poids de la couche de surface (5).

2. Article permettant la transmission d'une lumière laser selon la revendication 1, dans lequel une surface rugueuse (1a) est formée sur ledit organe (1, 30) permettant une transmission et ladite couche de surface (5) est prévue sur ladite surface rugueuse (1a).

3. Procédé de fabrication d'un article (10) permettant la transmission d'une lumière laser en formant une couche de surface (5) sur un organe (1, 30) permettant la transmission d'une lumière laser, caractérisé par le fait qu'il comprend l'étape d'amenée dudit organe (1, 30) permettant une transmission en contact avec une dispersion comprenant des particules (4) permettant la transmission d'une lumière laser, d'un troisième type, des particules (3) absorbant la lumière laser, d'un premier type, et des particules de dispersion de lumière laser (2), d'un deuxième type, qui ont un indice de réfraction supérieur à celui dudit organe (1, 30) permettant une transmission et dans lequel les particules (2) du deuxième type A constituent 70 à 20 % en poids de la couche de surface (5), les particules (3) du premier type C constituent 70 à 10 % en poids de la couche de surface (5), et les particules (4) du troisième type B constituent 20 à 50 % en poids de la couche de surface (5).

4. Procédé selon la revendication 3, dans lequel lesdites particules (4) permettant la transmission d'une lumière laser ont un point de fusion identique ou inférieur à celui dudit organe (1, 30) permettant une transmission et cet organe (1, 30) permettant une transmission est cuit avec la dispersion à une température supérieure au point de fusion desdites particules (4) permettant une transmission et à une température limite permettant audit organe (1, 30) permettant une transmission de conserver sa forme.

8

Fig. 1

Fig. 10

Fig. 2

Fig. 8

Fig. 3

Fig. 4

Fig. 5

Fig. 6

Fig. 7

Fig. 9